# EUROPEAN PATENT APPLICATION

(11) **EP 1 182 252 A1**
(43) Date of publication of application: **27.02.2002**
(21) Application number: 00925604.1
(22) Date of filing: 10.05.2000
(51) Int. Cl.: C12N 15/00, C12Q 1/68, C12M 1/34

(54) **METHODS FOR CONSTRUCTING DNA LIBRARY AND SUPPORT CARRYING DNA LIBRARY IMMOBILIZED THEREON**

(30) Priority: 10.05.1999 JP 12794399
(71) Applicant: Toyo Kohan Co., Ltd., Tokyo 102-8447 (JP); Nihon Parkerizing Hiroshima Co., Ltd., Hiroshima-shi, Hiroshima 734-0013 (JP)
(72) Inventor: TAKAHASHI, Kojiro, Hiroshima-shi, Hiroshima 734-0015 (JP); TAKAI, Osamu, Nihon Parkerizing Hiroshima Co., Ltd, Hiroshima-shi, Hiroshima 734-0003 (JP); TANGA, Michifumi, Toyo Kohan Co., Ltd., Kudamatsu-shi, Yamaguchi 744-8611 (JP)
(74) Representative: VOSSIUS & PARTNER
(86) International application number: PCT/JP00/03000
(87) International publication number: WO 00/68368

(57) **Abstract**

A purpose of the present invention relate to provide a method for constructing an original support on which a DNA (Deoxyribonucleic Acid) library and others are immobilized by utilizing micro amount of DNA test material, a method for constructing its replica supports and a support on which a duplicated DNA piece is immobilized in a molecular biological technology and/or a biochemical technology such as a gene technology, a protein technology, a cell technology and immunology. In a method for constructing a cDNA (complemetary DNA) library according to the present invention, Reverse Transcriptase enzyme is affected so as to immobilize the complementary DNA on a support after hybridizing mRNA (messenger RNA). Alternatively, mRNA is dehybridized from a cDNA library immobilized on a support and then the same cDNA library is immobilized on a support by utilizing the mRNA. In a method for constructing a genomic DNA (gDNA) library, a double stranded gDNA is ligased, the gDNA library is immobilized on a support. Alternatively, after the ligation of the double stranded gDNA, a sense portion of the gDNA is immobilized on a support. After dehybridizing an anti-sense portion of the gDNA library, the anti-sense portion is immobilized on a support.

## Description

### TECHNICAL FIELD

An industrial field of the present invention relates to a molecular biological technical and a biochemical technical such as a gene technology, a protein technology, a cell technology and an immunology technology, particularly to a method for constructing an original support on which a Deoxyribonucleic Acid (hereinafter, it is referred as "DNA") library is immobilized by utilizing micro amount of DNA test material, a method for constructing its replica supports and a support immobilized with a DNA piece.

### BACKGROUND OF THE INVENTION

In a conventional art, in the case of experimenting DNA, DNA is amplified by utilizing a Polymerase Chain Reaction (hereinafter, it is referred as "PCR") and divided to small groups since DNA is very important test material. The DNA test material is preserved at a remarkably low temperature in a freezer. In a conventional art, a DNA library is produced in a solution condition so that a replica of the DNA library can not be produced. Accordingly, it has to treat a DNA library test material in a solution condition obtained from micro amount of tissue or cells very carefully in order to search and diagnose its gene. A purpose of the present invention is to provide a method for constructing a DNA library support (original support) on which A DNA library is immobilized by utilizing micro amount of the DNA library test material. Another purpose of the present invention is to provide a method for constructing necessary number of replica supports.

Further, another purpose of the present invention is to provide a support on which a replica DNA piece is immobilized.

### DISCLOSURE OF THE INVENTION

In a method for constructing a cDNA (complementary DNA) library according to the present invention, the method is characterized of hybridizing oligo (dT) ₙ on a support and mRNA (messenger Ribosenucleic Acid, hereinafter, it is referred as "mRNA"), and affecting with RT (Reverse Transcriptase,hereinafter, it is referred as "RT" ) in order to immobilize complementary DNA.

In a method for constructing a cDNA library according to the present invention, mRNA is dehybridized from a cDNA library immobilized on a support. The method is characterized of immobilizing the same cDNA library by utilizing the dehybridized mRNA.

In a method for constructing a gDNA (genomic DNA) library according to the present invention, the method is characterized of ligasing a double stranded gDNA to oligo nucleotide on a support with restrictive enzyme portion.

In a method for constructing single stranded gDNA library according to the present invention, the method is characterized of utilizing a sense portion of a gDNA immobilized on the support as recited in claim 3.

In a method for single stranded gDNA library according to the present invention, the method is characterized of dehybridizing an anti-sense portion of the gDNA library as recited in claim 3 and synthetic immobilizing a sense portion on a support by utilizing the anti-sense portion.

In any methods according to the present invention as recited in one of claims 1 to 5, it is characterized that a support is previously chemical modificated with nucleotide or oligo nucleotide.
A substrate according to the present invention is characterized in that a DNA library is immobilized by any methods as recited in one of claims 1 to 6.

A support according to the present invention is characterized in that single stranded DNA library is immobilized.

### BRIEF DESCRIPTION OF THE DRAWINGS

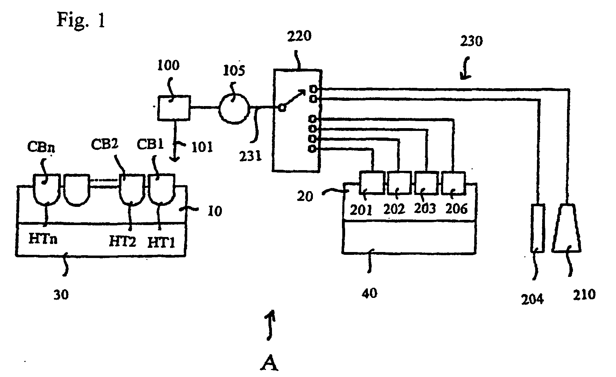

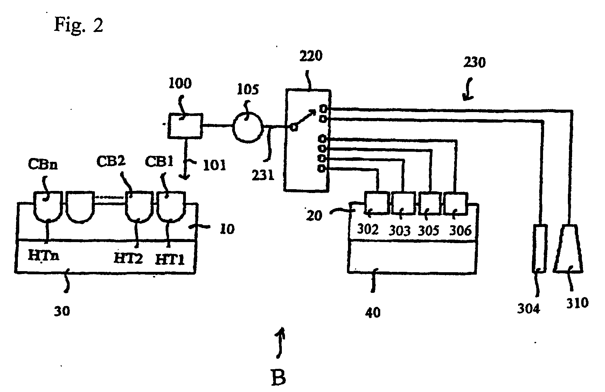

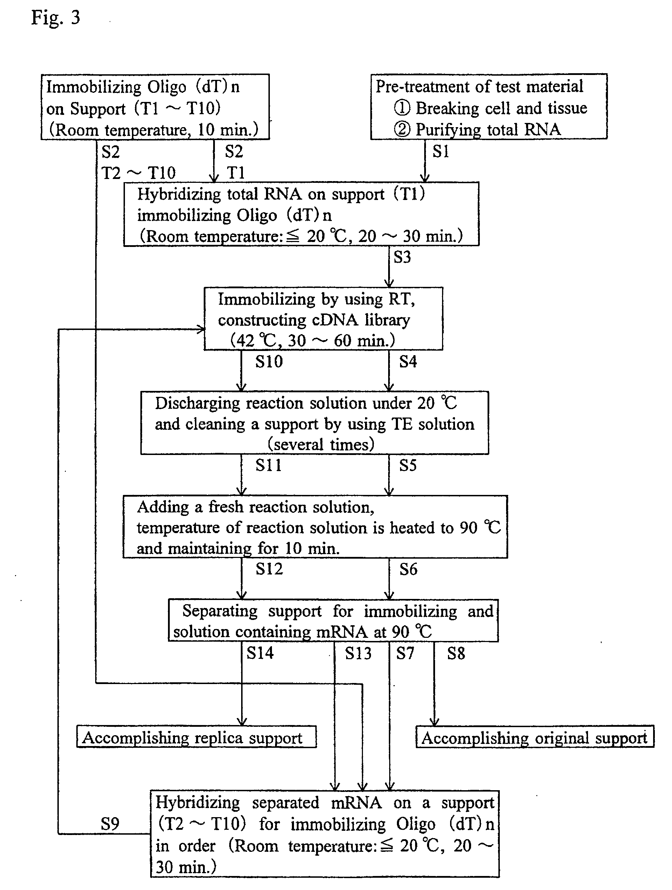

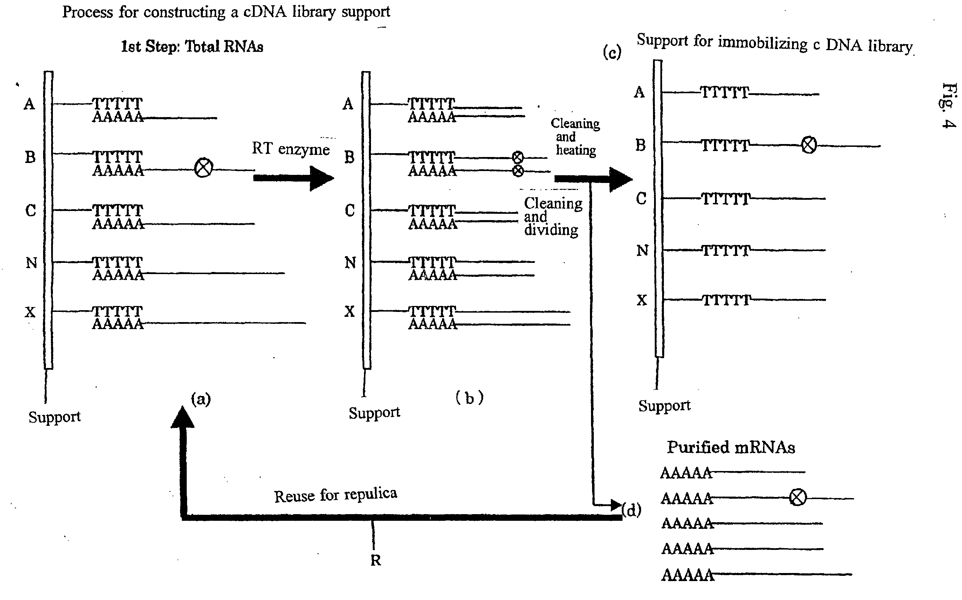

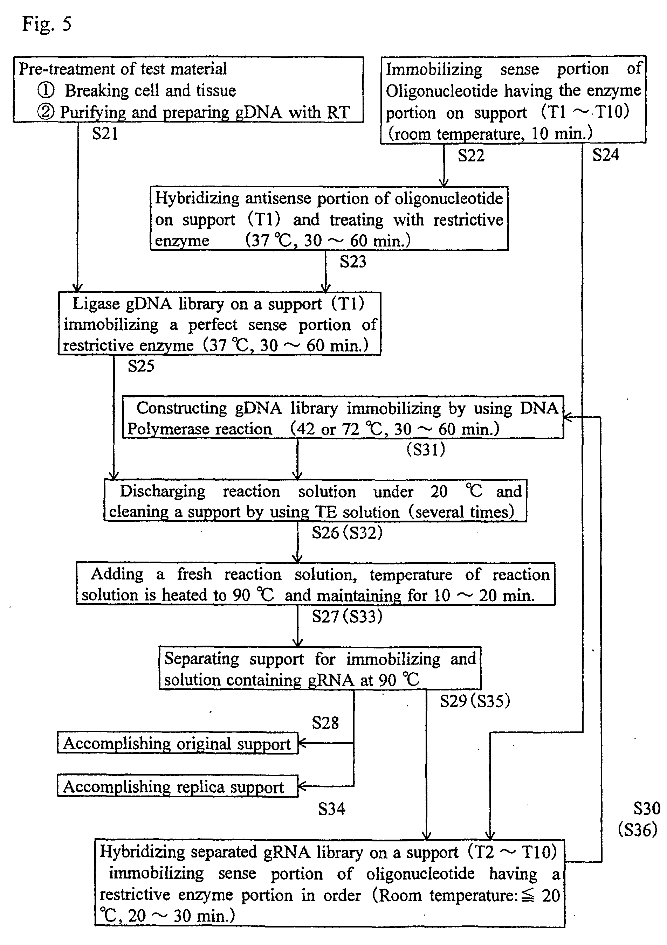

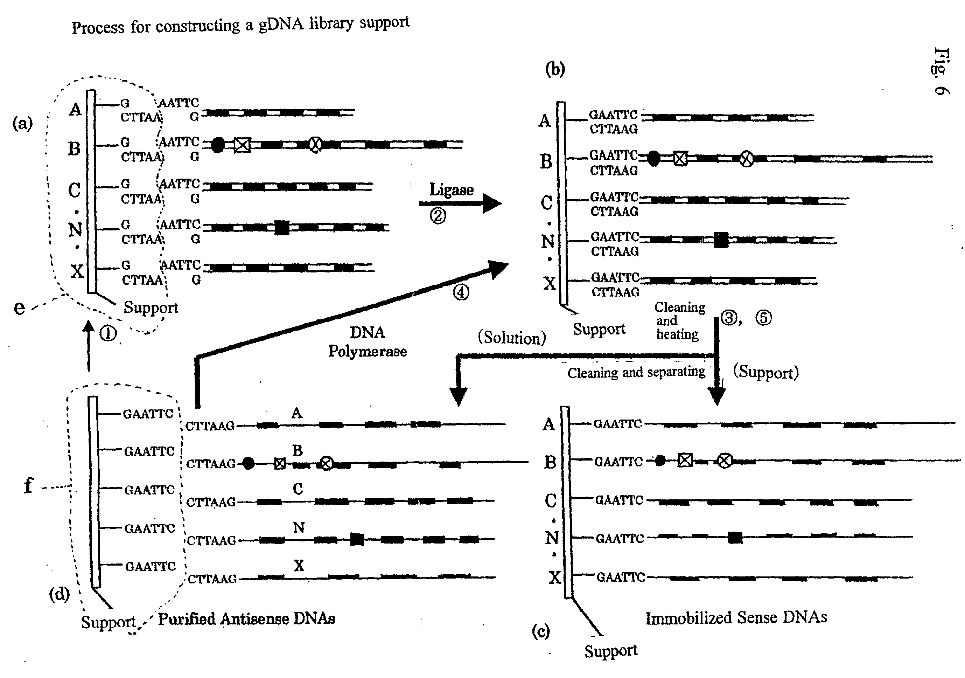

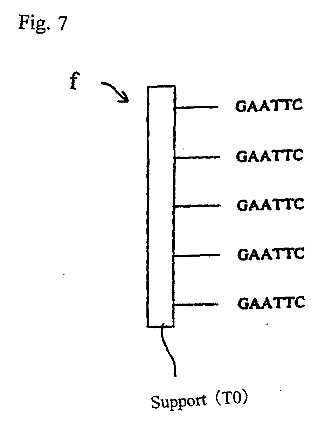

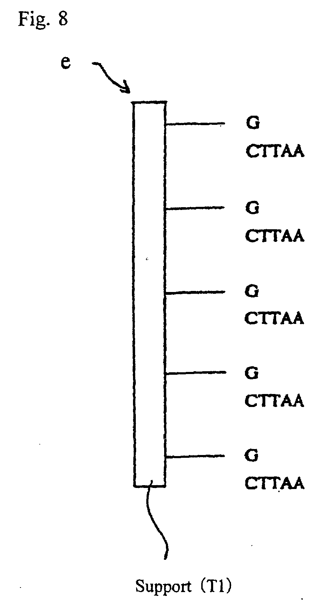

Fig. 1 shows a schematic view of a device for constructing a cDNA library support according to the present invention. Fig. 2 shows a schematic view of a device for constructing a gDNA library according to the present invention. Fig. 3 shows a flow chart for explaining a process for constructing a cDNA library support according to the present invention. Fig. 4 shows a schematic view for explaining a process for constructing a cDNA library support according to the present invention. Fig. 5 is a flow chart for explaining a process of constructing a gDNA library support according to the present invention. Fig. 6 is a schematic view for showing a process for constructing a gDNA library support according to the present invention. Fig. 7 shows an enlarged view of a support f. Fig. 8 shows an enlarged view of a support e.

### BEST MODE FOR CARRYING OUT THE INVENTION

An original support chemical modificated with respect to only nucleotide or oligonucleotide and a plurality of supports for a replica use are prepared so as to produce an original support or replica supports. The supports are set in a replica constructing device. A device for constructing a DNA library support according to the present invention will be explained with reference to accompanying drawings. Fig. 1 is a schematic view of a device for constructing a cDNA library support. Fig. 2 is a schematic view of a device for constructing a gDNA library support. Fig. 3 is a flowchart for explaining a process of constructing a cDNA library support. Fig. 5 is a flowchart for explaining a gDNA library support.

Fig. 1 shows a schematic view of a device for automatically constructing and duplicating a cDNA library support. A device A for constructing a DNA library support as shown in Fig. 1 comprises liquid feeding means 105 for feeding reaction solution to a container, liquid feeding switch means 220 for stopping the reaction solution and feeding new reaction solution, nozzle driving means 100 for driving a nozzle 101 for inleting/outleting test material in a front-rear and right-left direction in a plane and an upper-lower direction, solution temperature controlling means 30 for heating/cooling reaction solution in the container, test material container holding means 20 for holding containers in which each test material and/or solution are set for constructing a respective immobilized DNA library supports, and test material container temperature control means 40 for controlling the test material container holding means at a predetermined temperature and so on. It is preferable that the container holding means 10 can hold 96 test material containers or more than test material containers in view of a connection to a PCR device and/or a PCR product analysis device in near future. It is preferable that the test material comprises at least four test material container inserting holes forreplica supports. It is preferable that umber of the container inserting holes provided at the test material container holding means 20 is 10 holes or more than holes in view of a connection to the PCR device and/or the PCR product analysis device in near future. It is preferable that the container holding means 10 and the test material container holding means 20 are an aluminum block with good conductivity in view of thermally controlling the solution temperature controlling means 30 and the test material container temperature control means 40.

It is preferable that a support for constructing a DNA library support is a plate shape, a ball shape, a cube shape or a grain shape in the both cases of an original support and replica supports. Although a material of the support is not specified, it is preferable that material does not react with reaction solution or material does not deposit harmful material with respect to a DNA immobilization reaction. For example, plastic, glass, silicone and metal are preferable material. The plate shape, the ball shape, the cube shape and so on are preferable. Particularly, a support made of diamond or carbon atoms including diamond is preferable.

### (Production of Original support and its Replica support with cDNA Library)

With reference to Fig. 1 and Fig. 3, it will be explained a process for constructing an original support on which a cDNA library is immobilized and its replica supports. At first, necessary number (T1∼Tn) of supports of 3 mm x 3 mm x 0.1 mm chemically modificated with respect to oligo(dT) n (n is from 15 to 30) are prepared. These supports are chemically modificated with respect to only oligo (dT) n and a DNA library has not been immobilized on the supports. A reason why a support chemically modified with oligo (dT) n is used is easy to hybridize mRNA in Total RNA chemically modificated. These supports are inserted into containers CB1 to CBn and the containers are set in the container holding means 10. In such a case, it is preferable to insert one support into a first container in view of certainly constructing an original support as an immobilized cDNA library and its replica supports by utilizing micro amount of mRNA obtained from a small amount of test material. Regarding an order of setting containers CB1∼CBn into which a support chemically modificated is inserted at the container holding means 10, the container CB1 for an original support in which a support T1 chemically modificated is set is inserted into a first inserting portion HT1. Necessary number of containers (CB2∼CBn) for replica supports into which the corresponding number of supports chemically modificated (T2∼Tn) is set, respectively are inserted into a second inserting hole HT2, ... a n^{th} inserting hole HTn in order.

### (Production of Original support)

Reaction solution 203 including purified total RNA solution 201, RT enzyme solution 202 and nucleotide (dT, dA, dG, dC) is set in the test material container holding means 20 controlled at a predetermined temperature (i.e. 4 °C ). Tris-Ethylene-diamine-tetraacetic-acid (hereinafter, it is referred as "TE") solution 204 for cleaning/eluting DNA (buffer solution including Tris-Ethylene-diamine-tetraacetic acid) and a waste solution tank 210 and others are provided. A capillary tube 230 is provided as a liquid feeding path by connecting to the liquid feeding switch means 220 for liquid feeding the respective solution. It is preferable that the capillary tube 230 is an anti-corrosion stainless tube for liquid chromatography. It is preferable that a connection between the test material inlet nozzle 101 and the liquid feeding switch means 220 through the liquid feeding means 105 is a silicone tube 231. Then, the test material inlet nozzle 101 is moved to a position of the whole HT1 in the container holding means 10 so as to insert the nozzle 101 into the container CB1 in which the first support (T1) is set. The liquid feeding switch means 220 is provided at a side of the reaction solution and the reaction solution is inleted to the container CB1 by driving the liquid feeding means 105. The liquid feeding switch means 220 is shifted to the total RNA solution 201 and the predetermined amount of the solution 201 is inleted by the liquid' feeding means 105. After passing a predetermined time (for example, 20 to 30 minutes) at a temperature equal or lower than the predetermined temperature (for example 20°C), the liquid feeding switch means 220 is shifted to RT enzyme solution (enzyme 1) 202 so as to inlet a predetermined amount of the solution 202 by driving the liquid feeding means 105. After removing the test material inlet nozzle 101 from the container CB1, a temperature of the container holding means 10 is set at the predetermined temperature (for example, 42 °C), RT enzyme reaction for constructing cDNA from mRNA is proceeded by maintaining for a predetermined time (for example 30 to 60 minutes). After setting a temperature of the container holding means 10 at a temperature equal or lower than the predetermined temperature (for example, 20°C), the liquid feeding switch means 220 is shifted to the waste liquid tank 210 so as to discharge reaction solution in the container CB1 to the waste liquid tank 210 by driving the liquid feeding means 105. The liquid feeding switch means 220 is shifted to the TE solution 204 so as to inlet a predetermined amount of the TE solution 204 into the container CB1 by driving the liquid feeding means 105. A temperature of the container holding means 10 is heated to a predetermined temperature (for example 90 °C) by driving the solution temperature control means 30 so as to hybridize mRNA. Then, the liquid feeding switch means 220 is shifted to a container 206 for temporally preserving mRNA, dehybridized mRNA solution is moved to the container 206 for temporally preserving mRNA by driving the liquid feeding means 105.

### (Production of Replica supports)

In the next, it will be described a method for constructing replica supports by re-using mRNA dehybridized from the original cDNA library support produced by the above described method. At the first, after removing the test material inlet/outlet nozzle 101 from the container CB1, the nozzle 101 is moved to a container CB2 in which a replica support (T2) and mRNA solution 206 temporally preserved is inlet to the container CB2 by reversely driving the liquid feeding means 105. Then, steps explained for the production of the above original support are repeated. However, a latter step for inleting the Total RNA solution 201 can be omitted. The liquid feeding switch means 220 is provided at a side of reaction solution 203 of the container CB2. The reaction solution is inleted to the container CB2 by driving the liquid feeding means 105. After maintaining the container CB2 for a predetermined time (for example, 20 to 30 minutes) at a temperature equal or lower than a predetermined temperature (for example, 20 °C), the liquid feeding switch means 220 is shifted to the RT enzyme solution (enzyme 1) 202 so as to inlet a predetermined amount of the solution by driving the liquid feeding means 105. After removing the test material inlet/outlet nozzle 101 from the container CB2, a temperature of the container holding means 10 is maintained at a predetermined temperature (for example, about 42 °C) for a predetermined time (for example, 30 to 60 minutes) . After controlling a temperature of the container holding means 10 at a temperature equal or lower than a room temperature (20 °C), the liquid feeding switch means 220 is shifted to the waste liquid tank 210. The test material inlet/outlet nozzle 101 is inserted into the container CB2, reaction solution in the container CB2 is discharged to the waste solution tank 210 by driving the liquid feeding means 105. The liquid feeding switch means 220 is shifted to the TE solution 204 so as to inlet a predetermined amount of the TE solution 204 into the container CB2 by driving the liquid feeding means 105. Then, the liquid feeding switch means 220 is shifted to the waste liquid tank 210 so as to discharge the TE solution in the container CB2 into the waste liquid tank. By repeating the above process several times (equal or more than 5 times preferably), a first replica support duplicated from the original cDNA library support is produced. Necessary numbers of replica supports are produced by repeating a cyclic operation for constructing the replica support necessary times. Fig. 2 is a schematic view of a device automatically constructing and duplicating a gDNA library support. The device for constructing a DNA library support as shown in Fig. 2 comprises a liquid feeding means 105 for liquid feeding reaction solution and so on to a container, a liquid feeding switch means 220 for switching the liquid feeding of the reaction solution, a nozzle driving means 100 for driving a test material inlet/outlet nozzle 101 in a front-rear direction and left-right direction in a plane and an upper-lower direction, a container holding means 10 for holding a container in which a support is set, a container solution temperature control means 30 for heating/cooling the reaction liquid in the container, a test material container holding means 20 for holding containers in which test materials and test solutions for duplicating an immobilized DNA library support are set, respectively and a test material container temperature control means 40 for controlling the test material container holding means at a predetermined temperature. It is preferable that 96 test material containers or more than test material containers can insert to the container holding means 10 in view of connecting to a PCR device and/or a PCR product analysis device in near future. It is preferable that the test material container holding means 20 comprises at least four holes for a test material container in order to produce replica supports. It is preferable that a number of the test material holes provided at the test material container holding means 20 is equal or more than 10 in view of connecting to a PCR device and/or a PCR product analysis device in near future. It is preferable that the container holding means 10 and the test material container holding means 20 are made of aluminum with good thermal conductivity in view of thermally controlling the container liquid temperature control means 30 with Peltier element.

### (Production of Original support of gDNA Library and Its Replica supports)

With reference to Fig. 2 and Fig. 5, it will be explained a production of an original support immobilized with a gDNA library and its replica supports. Necessary number (T1∼Tn) of supports (for example, 3 mm x 3 mm x 0.1 mm) chemically modificated with oligo nucleotide (sense portion) having a restrictive enzyme portion are prepared. With respect to the original support (T1), oligo nucleotide (anti-sense portion) is hybridized and treated with restrictive enzyme so as to prepare a complete restrictive enzyme portion. The original support T1 is set in a container CB1 and supports T2∼Tn (replica supports) chemically modificated with oligo nucleotide (sense portion) having restrictive enzyme portion are set in containers CB2∼CBn. These containers are set in the solution holding means 10. Regarding a setting order, the container CB1 in which an original support T1 is set is inserted into a first inserting hole HT1 at the first and a second container and the successive containers CB2∼CBn in which each replica support is set are inserted in order. Reaction solution 303 including purified gDNA library solution 306 treated with restrictive enzyme, DNA Ligase solution (enzyme 1) 305, DNA Polymerase solution (enzyme 2) 302 and nucleotide (dT, dA, dG, dC) 303 is set in a test material solution holding means 20 of which a temperature is fixed at a predetermined temperature (24 °C). TE solution for cleaning/eluting DNA 304 and a waste liquid tank 310 are provided. A capillary tube 330 for the respective solution is connected to a liquid feeding switch means 220. It is preferable that the capillary tube 230 is an anti-resistant stainless tube for liquid chromatography. The liquid feeding switch means 220 and the test material inlet/outlet nozzle 101 and others are connected to a front end of the capillary tube 230 through the liquid feeding means 105. A silicone tube 231 is preferable for its connection. A test material inlet/outlet nozzle 101 is moved to a location of the hole HT1 of the container holding means 10 so as to insert the nozzle 101 into the container CB1 in which the first support (T1) is set. The liquid feeding switch means 220 is shifted to the reaction solution 303 so as to inlet a predetermined amount (for example, 17 µ L) of the reaction solution 303 by driving the liquid feeding means 105. The liquid feeding switch means 220 is shifted to the gDNA library solution 306 treated with restrictive enzyme so as to inlet a predetermined amount (for example, 2 µ L) of the solution 306 by driving the liquid feeding means 105. After maintaining the container CB1 at a temperature equal or lower than a predetermined temperature (for example, 20 °C) for a predetermined time (for example, 20 to 30 minutes), the liquid feeding switch means 220 is shifted to DNA Ligase solution (enzyme 1) 305 so as to inlet a predetermined amount (for example, 1 µ L) of the solution 305 into the container CB1 by driving the liquid feeding means 105. After removing the test material inlet/outlet nozzle 101 from the container CB1, a temperature of the container holding means 10 is controlled at a predetermined temperature (for example, 37°C). After maintaining the container CB1 for a predetermined time (for example, 30 to 60 minutes), the gDNA library immobilized with DNA Ligase is produced on the support T1. After controlling a temperature of the container holding means 10 at a predetermined temperature (for example, equal or less than 20 °C), the liquid feeding switch means 220 is shifted to the waste liquid tank 310 so as to insert the test material inlet/outlet nozzle 101 to the container CB1 and discharge the reaction solution in the container CB1 by driving the liquid feeding means 105. The liquid feeding switch 220 is shifted to the TE solution 304, a predetermined amount (for example, 500 µ L) of the TE solution is inleted to the container CB1 by driving the liquid feeding means 105. Then, the liquid feeding switch means 220 is shifted to the waste liquid tank 310 so as to discharge the TE solution in the container CB1. By repeating the process several times (for example, equal or more than 5 times), the immobilized support T1 is cleaned. After cleaning the immobilized support T1, the liquid feeding switch means 220 is shifted to the reaction solution 303 so as to inlet a predetermined amount (for example, 19 µ L) of the reaction solution 303 into the container CB1 by driving the liquid feeding means 105. By heating a temperature of the container holding means 10 at a predetermined temperature (for example, 90 °C) and maintaining the container CB1 for a predetermined time (for example, 10 to 20 minutes), anti-sense portion is dehybridized from the immobilized gDNA library. Then, the liquid feeding switch means 220 is shifted to the container 306 in which a gDNA library (anti-sense portion) is temporally preserved so as to outlet the gDNA library (anti-sense portion) solution from the container CB1. In the present stage, a production of the first gDNA library (sense portion) support (original support) is accomplished.

### (Production of Replica support)

After removing the test material inlet/outlet nozzle 101 from the container CB1, the nozzle 101 is moved to the next container CB2 in which a replica support (T2) is set so as to inlet the gDNA library (anti-sense portion) solution 306 temporally preserved into the container CB2. In order to produce a replica support, the above described cyclic operation is repeated necessary times so as to produce necessary number of replica supports. However, in the second embodiment described below, it is noted that DNA Polymerase solution (enzyme 2) 302 is selected during a replica constructing process so as to inlet a predetermined amount (for example, 1 µ L) of the solution 302 into the container CB2 by driving the liquid feeding means 105.

### Embodiments

### (Embodiment 1)

With reference to Fig. 1, Fig. 3 and Fig. 4, it will be explained a production of an original support immobilized with a cDNA library and its replica supports. Regarding pre-treatment of the test material, the test material is prepared by (1) breaking cell and tissue and purifying total RNA (see a step S1 in Fig. 3). Regarding a pre-treatment of a support immobilized with a cDNA library, rat's liver tissue of about 5 mm x 5 mm is homogenized in a test material kit (for example, ISOGEN sold by K.K. Nippon Gene) and the total RNA is purified in accordance with its protocol. Ten supports (T1∼T10) of 3 mm x 3 mm x 0.1 mm chemically modificated with oligo (dT) n (n is from 15 to 30) are prepared (see the step S2 in Fig. 3). The supports (T1∼T10) immobilized with amino group on its surface are treated with activating dihydric carbonic solution. After cleaning with ethanol and distilled water in order, the supports are maintained in the oligo (dT) n solution for 10 minuets. Each support (T1∼T10) is inserted into a respective containers CB1∼CB10 individually, the containers CB1∼CB10 are set in the temperature controlling aluminum block 10. Reaction solution 203 including purified total RNA solution 201, RT enzyme solution 202 and nucleotide (dT, dA, dG, dC) are set in a low temperature test material aluminum block 20 controlled at 4 °C. TE solution (buffer liquid including Tris-Ethylene-diamine-tetraacetic-acid) for cleaning DNA and the waste liquid tank 210 are provided at an exterior side of the low temperature test material aluminum block 20. The total RNA solution 201, the RT enzyme solution 202, the reaction solution 203, the TE solution 204, the waste liquid tank 210 is connected to an automatic 8-ways switching valve 220 through capillary tubes 230, respectively. A test material inlet/outlet capillary needle 101 is moved to a location of the inlet hole HT1 of the temperature control container aluminum block 10 so as to insert the capillary needle 101 into the container CB1 in which the first support T1 (original support) is set. The automatic 8-ways switching valve 220 is shifted to the reaction solution 203 so as to inlet the reaction solution 203 of 17 µ L into the container CB1 by driving a peristaltic pump 105. The automatic 8-ways switching valve 220 is shifted to the Total RNA solution 201, the solution 201 of 2 µ L is pumped by the peristaltic pump 105. In order to hybridize oligo (dT) immobilized on a surface of the support and mRNA in the Total RNA solution, the solutions are maintained at 20°C for 20 minutes (see a step S3 in Fig. 3 and Fig. 4(a)). After passing the time, the automatic 8-ways switching valve 220 is shifted to the RT enzyme solution 202 of 1 µ L so as to pump the RT enzyme solution 202 by the peristaltic pump 105. After removing the test material inlet/outlet capillary needle 101 from the container CB1, the container holding means 10 is controlled at 42 °C for 30 minutes so as to produce a cDNA library immobilized on the support T1 (immobilized support) by the RT enzyme (see a step S4 in Fig. 3 and Fig. 4 (b)). After cooling the container holding means 10 to 20 °C again, the automatic 8-ways switching valve 220 is shifted to the waste liquid tank 210 so as to insert the test material inlet/outlet capillary needle 101 into the container CB1. The reaction solution in the container CB1 is discharged to the waste liquid tank 210 by driving the peristaltic pump 105. The automatic 8-way switching valve 220 is shifted to the TE solution 204. The TE solution 204 of 500 µ L is inleted to the container CB1 by driving the peristaltic pump 105. Then the automatic 8-ways switching valve 220 is shifted to the waste liquid tank 210 so as to discharge the TE solution in the container CB1 to the waste solution tank 210. By repeating the operation several times (five time or more than times), the immobilized support T1 is cleaned (see a step S5 in Fig. 3). After cleaning the immobilized support T1, the automatic 8-ways switching valve 220 is shifted to the reaction solution 203, the reaction solution 203 of 19 µ L is inleted to the container CB1 by proving the peristaltic pump 105. The container holding means 10 is heated to 90°C, mRNA is dehybridized from the immobilized cDNA library after maintaining for 10 minutes (see a step S6 in Fig. 3 and Fig. 4 (d)). In the next, the automatic 8-ways switch valve 220 is shifted to the container 206 in which mRNA is temporally preserved, dehybridized mRNA solution is eluted and outleted from the container CB1 and preserved in the container 206 temporally (see a step S7 in Fig. 3). In accordance with the above steps, the first cDNA library support (original support) immobilized with a cDNA library is produced (see a step S8 in Fig. 3 and Fig. 4 (c) ) . After removing the test material inlet/outlet capillary needle 101 from the container CB1, the capillary needle 101 is moved to the container CB2 in which a replica support (T2) is set. The replica support (T2) is previously chemically modificated. The automatic 8-ways switching valve 220 is shifted to the container 206 for temporally preserving mRNA, mRNA of 19 µ L temporary preserved is inleted to the container CB2 by driving a peristaltic pump 105 (see an arrow R as shown in Fig. 4 (d) to Fig. 4 (a)). In order to hybridize immobilized oligo (dT) and mRNA, the container CB2 is maintained at 20 °C for 20 minutes. The automatic 8-ways switching valve 220 is shifted to the RT enzyme solution 202, the RT enzyme solution 202 of 1 µ L is inleted to the container CB2 by driving the peristaltic pump 105 (see a step S9 in Fig. 3). After removing the test material inlet/outlet capillary needle 101 from the container CB2, the container holding means 10 is controlled at 42°C for 30 minutes so as to produce a cDNA library support immobilized on the support (T2) by RT enzyme (see a step S10 in Fig. 3 and Fig. 4 (b) ) . After cooling the container holding means 10 to 20 °C again, the automatic 8-ways switching valve 220 is shifted to the waste liquid tank 210. The test material inlet/outlet capillary needle 101 is inserted into the container CB2 so as to discharge the reaction solution in the container CB2 to the waste liquid tank 210 by driving the peristaltic pump 105. The automatic 8-ways switching valve 220 is shifted to the TE solution 204, the TE solution 204 of 500 µ L is inleted into the container CB2 by driving the peristaltic pump 105. Then, the automatic 8-ways switching valve 220 is shifted to the waste liquid tank 210 so as to discharge the TE solution in the container CB2. By repeating the above operation 5 times, the immobilized support T2 is cleaned (see a step S11 in Fig. 3). After cleaning the immobilized support T2, the automatic 8-ways switching valve 220 is shifted to the reaction solution 203, the reaction solution 203 of 19 µ L is inleted to the container CB2 by driving the peristaltic pump. In the next, the container holding means 10 is heated to 90 °C and maintained for 10 minutes so as to dehybridize mRNA from the immobilized cDNA library (see a step S12 in Fig. 3). In the next, the automatic 8-ways switching valve 220 is shifted to the container 206 for temporally preserving mRNA, dehybridized mRNA solution is separated, eluted from the container CB2 by driving the peristaltic pump 5 (see Fig. 4 (d)) and temporally preserved in the container 206 (see a step S13 in Fig. 3). In accordance with the above steps, a replica cDNA library support (replica support) is produced (see a step S14 in Fig. 3 and Fig.4 (c)). With respect to the containers CB3∼CB10 in which supports T3∼T10 is set, respectively, the similar process is operated. By repeating the above steps S9 through S14 in order, eight replica supports are produced in order. By utilizing supports (T1∼T10) immobilized with a cDNA library of rat's liver tissue, gene is amplified by a PCR device with respect to 18S rRNA. It is confirmed the cDNA library is immobilized by an electrophoresis device. As the result, it can be confirmed that the original support T1 and the replica supports T2∼T10 are produced as normal cDNA library supports.

### (Embodiment 2)

With reference to Fig. 2, Fig. 5, Fig. 6 and Fig. 7, it will be explained a production of an original support immobilized with a gDNA library and its replica supports. Regarding the pre-treatment of the test material, test materials are prepared by (1) breaking cell and tissue and (2) purifying and treating gDNA with restrictive enzyme (see a step S21 in Fig. 5). Ten supports chemically modificated with a sense portion of oligo nucleotide having base sequence of a target restrictive enzyme portion are prepared. A size of the support is about 3 mm x 3 mm x 0.1 mm (see a step S22 in Fig. 5). A concept of the support is shown as a support f surrounded with a breaking line in Fig. 6 (d). Fig. 7 is an enlarged view of the portion. By utilizing one support chemically modificated, an anti-sense portion of the oligo nucleotide is hybridized. The support is treated with restrictive enzyme so as to produce one support (T1) having a complete restrictive enzyme cut portion (see a step S23 in Fig. 5 and an arrow ① in Fig. 6 (d) to Fig. 6 (a)). A concept of the support is shown as a support e surrounded with a broken line in Fig. 6 (a) . Fig. 8 is an enlarged view of its portion. A container CB1 in which the support T1 is inserted and nine supports chemically modificated with a sense portion of oligonucleotide having the restrictive enzyme portion are set in the container holding means 10. As shown in Fig. 2, the reaction solution 303 including purified gDNA library solution 306 treated with restrictive enzyme, DNA solution (enzyme 1) 305, DNA Polymerase solution (enzyme 2) 302 and nucleotide (dT, dA, dG, dC) is set in a low temperature test material aluminum block 20 controlled at 4 °C. TE solution 304 for cleaning/eluting DNA and a waste liquid tank 310 are provided at an exterior side of the low temperature test material aluminum block 20. As shown in Fig. 2, the reaction solution 303 including the gDNA (genomic DNA) library solution 306, the DNA Ligase solution (enzyme 1) 305, the DNA Polymerase solution (enzyme 2) 302 and nucleotide (dT, dA, dG, dC), the TE solution 304 for cleaning/eluting DNA and the waste liquid 310 are connected to an automatic 8-ways switching valve 220 through a capillary tube 230, respectively. The test material inlet/outlet capillary needle 101 is moved to a location of the hole HT1 of the temperature control container aluminum block 10 so as to insert the capillary needle 101 into the container CBB in which the first support T1 (original support) is set. The automatic 8-ways switching valve 220 is shifted to the reaction solution 303, the reaction solution 303 of 17 µ L is inleted to the container CB1 by driving the peristaltic pump 105. The automatic 8-ways switching valve 220 is shifted to the gDNA library solution 306 treated with restrictive enzyme, the solution 306 of 2 µ L is pumped by the peristaltic pump. After maintaining at 20°C for 20 minutes, the automatic 8-ways switching valve 220 is shifted to DNA Ligase solution (enzyme 1) 305 so as to inlet the solution 305 of 1 µ L to the container CB1 by driving the peristaltic pump 105. After removing the test material inlet/outlet capillary needle 101 from the container CB1, a temperature of the container holding means 10 is controlled at 37 °C for 30 minutes so as to produce the gDNA library immobilized by DNA Ligase on the support T1 (see a step S25 in Fig. 5 and an arrow ② in Fig. 6 (a) to Fig. 6 (b) ) . After cooling the container holding means 10 to 20 °C, the automatic 8-ways switching valve 220 is shifted to the waste liquid tank 310 so as to discharge the reaction solution in the CB1 by driving the peristaltic pump 105. The automatic 8-ways switching valve 220 is shifted to the TE solution 304, the TE solution of 500 µ L is inleted to the container CB1 by driving the peristaltic pump 105. Then, the automatic 8-ways switching valve 220 is shifted to the waste liquid tank 310, the TE solution in the container CB1 is discharged. By repeating these steps five times or more, the support T1 is cleaned (see a step S26 in Fig. 5). After cleansing the support T1, the automatic 8-ways switching valve 220 is shifted to the reaction solution 303. The reaction solution of 19 µ L is inleted to the container CB1 by driving the peristaltic pump 105. The temperature control container aluminum block 10 is heated to 90 °C and maintained for 10 minutes, an anti-sense portion is dehybridized from double stranded of a sense portion and the anti-sense portion of immobilized DNA library (see a step S27 in Fig. 5 and an arrow ③ in Fig. 6 (b) and Fig. 6 (c)). The automatic 8-ways switching valve 220 is shifted to a container 306 for preserving temporally so as to elute the anti-sense portion of the gDNA library solution from the container CB1 by driving the peristaltic pump 105 (see a step S29 in Fig. 5 and an arrow in Fig. 6(b) to Fig. 6 (d)). On the other hand, the sense portion is only immobilized on the support (T1). Thus, the first support, that is, single stranded gDNA library support T1 (original support) is produced (see a step S28 in Fig. 5 and Fig. 6 (c)). After removing the test material inlet/outlet capillary needle 101 from the container CB1, the capillary needle 101 is moved to the container CB2 in which the support T2 is set. The reaction solution including nucleotide is added to the container CB2 preserved at 20°C. The gDNA library solution 306 including only anti-sense portion temporally preserved is inleted to the container CB2 and maintained for 20 minutes (see a step S30 in Fig. 5). In the next, DNA Polymerase is added, heated to 37 °C and maintained for one hour. As the result, double stranded g DNA library of which a sense portion is immobilized on the support T2 is produced (see a step S31 and an arrow ④ in Fig. 6 (d) to Fig. 6 (b) ) . The container CB2 in which the support T2 immobilized with the above double stranded gDNA library is set is controlled at 20 °C. After shifting the automatic 8-ways switching valve 220 to the waste liquid tank 310, the test material inlet/outlet the capillary needle 101 is inlet into the container CB2. The reaction solution in the container CB2 is discharged by driving the peristaltic pump 105. The automatic 8-ways switching valve 220 is shifted to the TE solution 304, the TE solution of 500 µ L is inleted to the container CB2 by driving the peristaltic pump 105. Then, the automatic 8-ways switching valve 220 is shifted to the waste liquid tank 310, the TE solution in the container CB2 is discharged. By repeating the above steps 5 times or more than times, the support T2 is cleaned (see a step S32 in Fig. 5) . After cleaning the support T2, the automatic 8-ways switching valve 220 is shifted to the reaction solution 303 so as to inlet the reaction solution of 19 µ L into the container CB2 by driving the peristaltic pump 105. The aluminum block 10 is heated to 90 °C and maintained for 10 minutes so as to dehybridize the anti-sense portion from immobilized gDNA library with double stranded of the sense portion and the anti-sense portion (see a step S33 in Fig. 5 and an arrow ⑤ in Fig. 6 (b) and Fig. 6 (c)). The automatic 8-ways switching valve 220 is shifted to a container for preserving temporary so as to outlet the anti-sense portion of gDNA library solution from the container CB2 (see a step S35 in Fig. 5 and an arrow in Fig. 6 (b) to Fig. 6 (d)) . A second single stranded gDNA library support, that is, a replica support T2 on which a sense portion is immobilized is produced (see a step S34 in Fig. 5 and Fig. 6 (c)). By repeating the above steps, double stranded gDNA library is immobilized on a support. An anti-sense portion is dehybridized from the double stranded immobilized gDNA library so as to produce remained number (T3∼T10) of single stranded gDNA library supports (replica supports). That is, a process including steps as shown in Fig. 6 (b), 6 (d), Fig. 6 (b) and Fig. 6 (d) is repeated so that any number of supports on which the same single stranded gDNA library is immobilized can be produced (see Fig. 6 (c)).

### POSSIBILITY OF USE IN THE INVENTION

In a device according to the present invention, a production of a support immobilized with cDNA library from mRNA and a gDNA library treated with restrictive enzyme of gDNA can be produced easily. Although it is impossible to produce replica supports duplicated from DNA library solution in a conventional art, necessary number (until mRNA and gDNA are chemically or physically broken) of replica supports are easily produced as immobilized DNA supports for a short time. An immobilized DNA library support and its replica supports can be produced by collecting micro amount of gene material from cultured cell or tissue of an important detected object at one time. With respect to the same kind of test materials, various kinds of gene research and detection can be operated. It is unaccountable benefit. By utilizing the immobilized DNA library support and its replica supports, budget and manual work for developing a new gene diagnosis technology would be remarkably saved in future. If blood is collected from a patient or tissue is collected in his medical operation at one time for gene diagnosing, re-use of the collected blood/tissue can be easily accomplished with respect to preventive medical research. Since a plurality of immobilized DNA library supports are produced semi-eternally. These facts can bring a big benefit by reducing mental and/or economic damage with respect to a patient as less as possible.

## Claims

1. A method for constructing a cDNA library immobilized with complementary DNA comprising:
a step of affecting RT (Reverse Transcriptase) enzyme after hybridizing mRNA and oligo (dT) on a support.

2. A method for constructing a cDNA library comprising:
a step of dehybridizing mRNA from a cDNa library immobilized on a support,
a step of immobilizing the same cDNA library on another support by utilizing said mRNA.

3. A method for constructing a gDNA library comprising:
a step of immobilizing a gDNA library after ligasing double stranded gDNA with respect to oligo nucleotide on a support having respective enzyme portion.

4. A method for constructing one gDNA library by utilizing an immobilized sense portion of said gDNA library on said support produced in claim 3.

5. A method for constructing single stranded gDNA library comprising:
a step of synthetically immobilizing a sense portion on a support by utilizing an anti-sense portion after dehybridizing said anti-sense portion of said gDNA library produced in claim 3.

6. A method as claimed one of claims 1 through 5, wherein said support is previously chemically modificated with nucleotide or oligo nucleotide.

7. A support immobilized with a DNA library produced in accordance with the method as claimed in one of claims 1 through 6.

8. A support immobilized with single stranded DNA library.
